# EUROPÄISCHE PATENTSCHRIFT

(11) **EP 1 169 119 B1**
(45) Veröffentlichungstag und Bekanntmachung des Hinweises auf die Patenterteilung: **15.10.2003**
(21) Anmeldenummer: 00926738.6
(22) Anmeldetag: 15.03.2000
(51) Int. Cl.: B01J 8/06, B01J 19/24, B01J 19/00

(54) **ROHRBÜNDELREAKTOR, INSBESONDERE FÜR KATALYTISCHE GASPHASENREAKTIONEN**
MULTI-TUBE FIXED-BED REACTOR, ESPECIALLY FOR CATALYTIC GAS PHASE REACTIONS
REACTEUR A FAISCEAU DE TUBES DESTINE NOTAMMENT A DES REACTIONS EN PHASE GAZEUSE CATALYTIQUE

(30) Priorität: 16.03.1999 DE 19912735; 23.11.1999 DE 19956329
(43) Veröffentlichungstag der Anmeldung: 09.01.2002
(73) Patentinhaber: BASF AKTIENGESELLSCHAFT, 67056 Ludwigshafen (DE)
(72) Erfinder: OLBERT, Gerhard, D-69221 Dossenheim (DE); CORR, Franz, D-67067 Ludwigshafen (DE); REUTER, Peter, D-68199 Mannheim (DE); WAMBACH, Ludwig, D-68723 Schwetzingen (DE); HAMMON, Ulrich, D-68165 Mannheim (DE)
(74) Vertreter: Kinzebach, Werner, Dr.
(86) Internationale Anmeldenummer: EP0002304
(87) Internationale Veröffentlichungsnummer: WO00054877

(56) Entgegenhaltungen:
- DE-A- 2 830 765
- US-A- 3 566 961
- US-A- 4 398 595
- US-A- 4 505 879
- US-A- 5 821 390

## Beschreibung

Die vorliegende Erfindung betrifft einen Rohrbündelreaktor und die Verwendung eines derartigen Reaktors zur Durchführung von katalytischen Gasphasenreaktionen, insbesondere zur Durchführung von exothermen und endothermen katalytischen Gasphasenreaktionen, wie der Herstellung von Phthalsäureanhydrid (PSA), Acrylsäure (AA), Methacrylsäure (MAA), Acrolein, Maleinsäureanhydrid (MSA), Glyoxal, Phosgen, Blausäure oder Vinylformamid (VFA).

Rohrbündelreaktoren werden in der chemischen Industrie meist zur Durchführung von katalytischen Gasphasenreaktionen an Festbettkatalysatoren verwendet.

Üblicherweise bestehen Rohrbündelreaktoren aus einem, in einem Mantel angeordneten, aus zahlreichen parallelen Reaktionsrohren aufgebauten Reaktionsrohrbündel. Die Reaktionsrohre, die üblicherweise geträgerte Katalysatoren enthalten, sind mit ihren offenen Enden in Rohrböden abdichtend befestigt und münden in jeweils eine am oberen bzw. unteren Ende mit dem Mantel verbundene Haube. Neben geträgerten Katalysatoren können die Reaktionsrohre, alternativ oder zusätzlich, Schalenkatalysatoren, Vollkatalysatoren, geordnete Packungen aus Katalysatormaterial, die einem statischen Mischer vergleichbar angeordnet sind, enthalten. Schließlich ist es auch möglich, die Innenwand der Reaktionsrohre mit Katalysatormaterial zu beschichten. Über die Hauben wird das die Reaktionsrohre durchströmende Reaktionsgemisch zu- bzw. abgeführt. Durch den zwischen dem obersten und untersten Rohrboden befindlichen, die Reaktionsrohre umgebenden Raum, der durch Umlenkbleche unterteilt sein kann, wird ein Wärmetauschmittelkreislauf geleitet, um Reaktionswärme zu- bzw. abzuführen. Dazu weist der Mantel des Rohrbündelreaktors Mittel zur Zu- und Abführung des Wärmetauschmittels auf, meist geeignete Ein- bzw. Austrittsringkanäle, durch die das Wärmetauschmittel mit Hilfe geeigneter Pumpen in einem Kreislauf geführt wird. Nach dem Verlassen des Rohrbündelreaktors wird das Wärmetauschmittel beispielsweise in einem außenliegenden Wärmetauscher wieder auf eine vorgegebene Solltemperatur gebracht, bevor es erneut in den Reaktor eintritt.

Bei exothermen Reaktionen kann auch mittels einer Siedekühlung temperiert werden.

Die im industriellen Produktionsprozeß verwendeten Rohrbündelreaktoren weisen einen Durchmesser von mehreren Metern auf. Aus wirtschaftlichen Gründen werden in den Reaktoren eine möglichst große Anzahl von Reaktionsrohren eingesetzt. Bei einem Reaktor von mehreren Metern Durchmesser liegt die Zahl der Reaktionsrohre meist im Bereich von 10.000 bis 50.000, bevorzugt im Bereich von 10.000 bis 30.000 Rohren. Bisher stand bei industriellen Rohrbündelreaktoren im Vordergrund, die Rohre so dicht wie möglich zu packen, um bei einer maximalen Reaktionsrohrzahl einen möglichst geringen Reaktordurchmesser zu erzielen. Üblicherweise sind die Rohre dabei in einer Dreiecksanordnung positioniert, meist in einem gleichseitigen Dreieck. Als Maß für die kompakte Anordnung der Reaktionsrohre dient das Verhältnis der sogenannten Rohrteilung *t* zum Außendurchmesser *d*_{*a*} eines Rohres. Unter Rohrteilung versteht man dabei den Abstand der zentrischen Innenachsen von zueinander nächstliegenden Reaktionsrohren. Bekannte industrielle Reaktoren, etwa der in den Ausführungsbeispielen der deutschen Offenlegungsschrift DE 44 31 957 A1 beschriebene Reaktor, weisen ein Verhältniss von Rohrteilung zu Rohraußendurchmesser von 1,28 oder weniger auf.

Insbesondere bei der Durchführung von stark exothermen Oxidationsreaktionen, wie der Herstellung von Phthalsäureanhydrid, Acrylsäure, Methacrylsäure, Acrolein, Maleinsäureanhydrid oder Glyoxal spielt die exakte Kontrolle der Reaktionstemperatur eine entscheidende Rolle. Bei diesen Reaktionen wird ein Gasgemisch durch die Reaktionsrohre geleitet, welche eine Festbettanordnung eines katalytisch aktiven Multimetalloxids enthalten. Beispielsweise werden Rohrbündelreaktoren zur Herstellung von Phthalsäureanhydrid eingesetzt, welches ein wichtiges Zwischenprodukt zur Herstellung von synthetischen Harzen, Phthalatweichmachern, Phthalocyaninfarbstoffen und weiteren Feinchemikalien ist. Die weltweite Produktion von Phthalsäureanhydrid beträgt mehr als 4.000.000 Tonnen pro Jahr. Nach dem wichtigsten Herstellungsverfahren wird Phthalsäureanhydrid heute größtenteils durch Gasphasenoxidation von o-Xylol mit Luft als Oxidans hergestellt. Dazu wird o-Xylol verdampft, mit einem Überschuß an Luft gemischt und bei 340 - 440 °C über den in den Reaktionsrohren befindlichen Katalysator geleitet. Der Katalysator kann beispielsweise aus einem Gemisch von V₂O₅ und TiO₂ mit Promotoren auf keramischen Körpern wie z. B. Porzellan- oder SiC-Kugeln oder -ringen bestehen. Typische Abmessungen dieser Keramikkörper betragen ca. 6 mm x 6 mm bzw. 8 mm x 6 mm. Das o-Xylol wird dabei mit einer Selektivität von 78 - 80 % zu Phthalsäureanhydrid oxidiert. Die Oxidation ist mit ca. - 1.110 kJ/mol stark exotherm.

Als Wärmetauschmittel eignen sich insbesondere Temperiermedien, die im bevorzugten Reaktionstemperaturbereich von 250 °C bis 500 °C, bevorzugt von 250 °C bis 380 °C flüssig sind. Besonders günstig ist beispielsweise die Verwendung von Schmelzen von Salzen wie etwa einer, insbesondere bei der PSA-Synthese bevorzugt eingesetzten Schmelze eines Gemischs aus Kaliumnitrat, Natriumnitrit und Natriumnitrat.

Die Verfahrensführung, insbesondere die Temperierung des Reaktors erfordert aus mehreren Gründen besondere Aufmerksamkeit: Bei der großen Anzahl von Röhren im Reaktor ist es erforderlich, daß sämtliche Rohre im gesamten Querschnitt mit der gleichen und zeitlich konstanten Gasmischung angeströmt werden, damit die Reaktion in allen Rohren gleich schnell und nicht in einigen bevorzugten Rohren besonders schnell abläuft. Insbesondere aber kann die hohe freiwerdende Reaktionsenthalpie dazu führen, daß der Katalysator bei Abweichungen vom vorgegebenen Temperaturbereich in einzelnen Rohren sintert oder schmilzt oder inaktiv wird. Dies ist mit beträchtlichen Risiken für die Anlage verbunden. Durch Inhomogenitäten in der Beaufschlagung werden außerdem die Reaktionsbedingungen in den Rohren unterschiedlich. Dadurch entstehen in erhöhtem Maß Nebenprodukte, welche die Ausbeute vermindern und in späteren Reinigungsstufen von dem gebildeten Phthalsäureanhydrid getrennt und entsorgt werden müssen. Die Reaktionstemperatur in Strömungsrichtung längs eines Reaktionsrohres duchläuft bei der Gasphasenoxidation ein Maximum, welches man als Hotspot (Heißpunkt) bezeichnet. Ein solcher Hotspot ist zwar grundsätzlich erwünscht. Problematisch ist jedoch eine zu hohe Hotspot-Temperatur, denn sie führt sowohl zu einer verringerten Lebensdauer des Katalysators als auch zu einer Abnahme der Selektivität der Reaktion.

Prinzipiell hat eine wirksame Reaktortemperierung daher die Aufgabe, Temperaturungleichverteilungen über den Querschnitt des Reaktors zu verringern und das Auftreten von unerwünscht hohen Hotspots zu verhindern.

Bei den bisherigen Reaktoren, die generell ein möglichst niedriges Verhältnis von Rohrteilung zu Rohraußendurchmesser aufweisen, war eine effektive Reaktortemperierung nur eingeschränkt möglich. Insbesondere bei zylindrischen Reaktorgeometrien wird das Wärmetauschmittel im Querstrom von einem Bereich außerhalb des Reaktionsrohrbündels zu einem reaktionsrohrfreien Innenraum des Reaktors bzw. umgekehrt geleitet. Dies führt zu einem starken Druckverlust und somit zu einem begrenzten Wärmetauschmittelstrom. Bisher war man daher gezwungen, leistungsfähige und folglich sehr teuere Pumpeinrichtungen zum Fördern des Wärmetauschmittels einzusetzen.

Aufgabe der vorliegenden Erfindung ist es nun, einen Reaktor zur Verfügung zu stellen, der eine gleichmäßigere Temperaturverteilung über den radialen Reaktorquerschnitt ermöglicht und zu hohe Wärmetauschmittelhotspots weitgehend verringert.

Gelöst wird diese Aufgabe durch den erfindungsgemäßen Rohrbündelreaktor mit den Merkmalen des vorliegenden Anspruchs 1. Erfindungsgemäß wird vorgeschlagen, bei größeren Reaktoren, bei denen aufgrund der zahlreichen Reaktionsrohre eine hohe abzuführende Reaktionswärme anfällt, das Verhältnis von Rohrteilung *t* zu Rohraußendurchmesser *d*_{*a*} vom Reaktordurchmesser bzw. vom Rohrbündelaußendurchmesser *d*_{*RBa*} abhängig zu machen. Insbesondere wird erfindungsgemäß vorgeschlagen, ein Verhältnis von Rohrteilung *t* zu Rohraußendurchmesser *d*_{*a*} von wenigstens 1,3 vorzusehen. Die Reaktionsrohre sind dabei vorzugsweise so angeordnet, daß drei benachbarte Reaktionsrohre ein, vorzugsweise gleichseitiges Dreieck bilden. Die Rohrteilung *t* entspricht in diesem Fall der Seitenlänge des Dreiecks.

Gegenstand der vorliegenden Erfindung ist daher ein Rohrbündelreaktor mit einem in einem Mantel angeordneten, aus zahlreichen parallelen Reaktionsrohren bestehenden Reaktionsrohrbündel und mit Mitteln zur Zu- und Abführung eines die Reaktionsrohre umströmenden Wärmetauschmittels, wobei die Reaktionsrohre einen Außendurchmesser *d*_{*a*} und eine Rohrteilung *t* aufweisen, wobei der Reaktor dadurch gekennzeichnet ist, daß das Verhältnis *t*/*d*_{*a*} von Rohrteilung zu Rohraußendurchmesser eines Reaktionsrohrs wenigstens 1,3 beträgt.

Mit dem erfindungsgemäßen Rohrbündelreaktor sind zahlreiche Vorteile verbunden.

Die Erhöhung des Verhältnisses von Rohrteilung zu Rohraußendurchmesser erlaubt bei einer gegebenen Pumpenleistung höhere Wärmetauschmittelströme und führt damit zu einer besseren Temperaturvergleichmäßigung über den Querschnitt und zu einer Verringerung des Wärmetauschmittelhotspots.

Überraschend stellt man fest, daß es möglich ist, die Wärmetauschmitteleintrittstemperatur anzuheben, ohne die maximal zulässige Austrittstemperatur des Wärmetauschmittels zu überschreiten. Dies führt zu einer verbesserten Selektivität der Reaktion und einer daraus resultierenden Erhöhung der Ausbeute. Die Kapazität des Reaktors kann so bis zu 2 % gesteigert werden.

Die Verringerung des Wärmetauschmittelhotspots führt zu einer größeren Betriebssicherheit, da die Gefahr des Zündens des Reaktionsgemisches stark verringert wird.

Speziell beim Einsatz des erfindungsgemäßen Rohrbündelreaktors bei der PSA-Synthese wurde gefunden, daß ein Teilungsverhältnis von mehr als 1,3 eine deutlich höhere Beladung des einströmenden Gasgemisches mit o-Xylol erlaubt.

Die gegenüber dem Stand der Technik nur geringfügig vergrößerte Rohrteilung führt auch nur zu einer geringen Vergrößerung des Reaktordurchmessers. Interessanterweise stellt man fest, daß diese geringe Zunahme der Abmessungen bei gegebener Pumpleistung einen nahezu doppelt so hohen Wärmetauschmittelstrom erlaubt.

Erfindungsgemäß wird ferner vorgeschlagen, daß das Verhältnis *t*/*d*_{*a*} von Rohrteilung zum Außendurchmesser eines Reaktionsrohrs mit steigendem Außendurchmesser *d*_{*RBa*} des Reaktionsrohrbündels ansteigt. Damit ist es möglich, der zu- bzw. abzuführenden Reaktionswärme Rechnung zu tragen, die mit steigendem Rohrbündelaußendurchmesser und damit stark ansteigender Rohrzahl sehr hoch wird.

Das erfindungsgemäß vorgeschlagene Teilungsverhältnis von mehr als 1,3 wird besonders vorteilhaft bei großen Reaktoren eingesetzt. Gemäß einer ersten bevorzugten Ausführungsform weist das Reaktionsrohrbündel einen im wesentlichen kreisförmigen Querschnitt mit einem Außendurchmesser *d*_{*RBa*} von mehr als 4 m auf. Ein solches Reaktionsrohrbündel hat meist einen rohrfreien Zentralbereich über den das radial an den Reaktionsrohren vorbeiströmende Wärmetauschmittel axial abfließen kann.

Besonders bevorzugt liegt bei dieser Ausführungsform das Verhältnis *t*/*d*_{*a*} von Rohrteilung zu Außendurchmesser eines Reaktionsrohrs bei Reaktionsrohrbündeldurchmessern zwischen 4 m und 12 m im Bereich von 1,3 bis 1,6 und bei Reaktionsrohrbündeldurchmessern zwischen 4 m und 10 m am bevorzugtesten im Bereich von 1,3 bis 1,5.

Rohrbündelreaktoren mit derartigen Durchmessern weisen im allgemeinen 10.000 bis 50.000, vorzugsweise 10.000 bis 30.000 Reaktionsrohre auf.

Die Erfindung ist jedoch nicht auf Reaktoren mit kreiszylindrischen Reaktionsrohrbündeln beschränkt. Falls beispielsweise Reaktionsrohrbündel mit einem rechteckigen Querschnitt oder einem kreisförmigen Querschnitt mit reaktionsrohrfreien Endsegmenten verwendet werden, ist das Verhältnis von Rohrteilung zu Rohraußendurchmesser bevorzugt von der vom Wärmetauschmittel quer durchströmten Tiefe *d*_{*RBt*} des Rohrbündels abhängig. Bei einem erfindungsgemäß vorgesehenen Teilungsverhältnis von 1,3 weist das Reaktionsrohrbündel bevorzugt einen im wesentlichen rechteckigen Querschnitt mit einer parallel zur Strömungsrichtung des Wärmetauschermittels gemessenen Rohrbündeltiefe von wenigstens 1,3 m auf.

Vorteilhaft liegt die Tiefe *d*_{*RBt*} des Reaktionsrohrbündels zwischen 1,3 m und 3 m, wobei dann das Verhältnis *t*/*d*_{*a*} von Rohrteilung *t* zum Außendurchmesser *d*_{*a*} eines Reaktionsrohrs im Bereich von mehr als 1,3 bis 1,6 liegt.

Üblicherweise sind die Reaktionsrohre aus ferritischem Stahl gefertigt und weisen eine typische Wanddicke von 1 bis 3 mm auf. Ihr Innendurchmesser beträgt in der Regel 20 bis 70 mm, bevorzugt 20 bis 35 mm. Die typische Länge der Reaktionsrohre, und damit die Länge des zylindrischen Bereichs des Reaktors liegt im Bereich von 1,5 bis 7 m.

Bei Reaktionsrohrbündeln mit im wesentlichen rechteckigem Querschnitt beträgt das Verhältnis von Querschnittstiefe *d*_{*RBt*} zu Querschnittslänge des Bündels bevorzugt 1:1 bis 1:10, besonders bevorzugt 1:1,5 bis 1:4.

Mit dem erfindungsgemäßen Reaktor können Durchflußmengen des Wärmetauschmittels, etwa einer Salzschmelze, von 10.000 bis 20.000 m³ pro Stunde realisisiert werden.

Mit einer Verringerung des reaktionsbedingten Hotspots im Reaktionsrohr wird einerseits die Lebensdauer des Katalysators verlängert und andererseits die Selektivität der Reaktion verbessert. Insbesondere die Verlängerung der Katalysatorlebensdauer stellt einen wichtigen Vorteil des erfindungsgemäßen Reaktors da, denn mit dem ab einer gewissen Betriebsdauer notwendigen Austausch des Katalysatormaterials sind hohe Kosten und lange Abschaltzeiten des Reaktors verbunden.

Häufig wird die Reaktionsführung in den Rohren des Reaktors über die Katalysatorfüllung gesteuert. Beispielsweise kann man eine strukturierte Schüttung von zwei unterschiedlich aktiven Katalysatoren verwenden und so in Längsrichtung des Reaktionsrohrs unterschiedliche Reaktionsbedingungen schaffen. Gemäß einer weiteren vorteilhaften Ausführungsform des erfindungsgemäßen Rohrbündelreaktors ist der Reaktorinnenraum, in Längsrichtung der Reaktionsrohre gesehen, in mindestens zwei Zonen unterteilt, die von verschieden temperiertem Wärmetauschmittel durchströmt werden. Durch diese Maßnahme wird einerseits eine weitere Möglichkeit der Reaktionsführung geschaffen, indem man die beiden Katalysaoren auf unterschiedliche Temperaturen thermostatisiert. Andererseits ist es bei manchen Prozessen sogar möglich, lediglich einen Katalysatortyp zur Füllung der Rohre zu verwenden und die Reaktionführung ausschließlich über zwei oder mehr, vorzugsweise bis zu fünf, unterschiedlich temperierte Zonen zu bewirken. Bevorzugt werden in aufeinanderfolgenden Zonen Temperaturunterschiede des Wärmetauschmittels von bis zu 60 °C, vorteilhaft bis 40 °C realisiert. Die Trennung der einzelnen Zonen voneinander wird bevorzugt durch Rohrbodenbleche gewährleistet, die im wesentlichen horizontal im Reaktor angeordnet sind und Ausnehmungen zur Durchführung der Reaktionsrohre besitzen.

Gegenstand der vorliegenden Erfindung ist auch die Verwendung eines erfindungsgemäßen Rohrbündelreaktors zur Durchführung von katalytischen Gasphasenreaktionen.

Gegenstand der vorliegenden Erfindung ist insbesondere die Verwendung eines erfindungsgemäßen Rohrbündelreaktors zur Durchführung von Oxidationsreaktionen, insbesondere zur Herstellung von Phthalsäureanhydrid, Maleinsäureanhydrid, Acrylsäure, Acrolein, Methacrylsäure, Glyoxal, Phosgen, Blausäure oder Vinylformamid. Derartige Herstellungsverfahren unter Verwendung von Rohrbündelreaktoren mit Katalysatoren in Festbettanordnung sind bekannt (vgl. beispielsweise Ullmann's Encyclopedia of Industrial Chemistry, 5^{th} Edition, Volume B4, Table 7 auf Seite 103, mit weiteren Nachweisen).

Im folgenden wird die Erfindung unter Bezugnahme auf in der beigefügten Zeichnung dargestellte, bevorzugte Ausführungsbeispiele näher erläutert. Die unten erwähnte Herstellung von Phthalsäureanhydrid wurde rein beispielhaft für die Verwendung des erfindungsgemäßen Reaktors bei oxidativen Gasphasenreaktionen herangezogen und stellt keine Beschränkung der Erfindung auf diesen Anwendungsfall dar.

In der Zeichnung zeigt:
- Figur 1: eine schematische Darstellung eines in eine Anlage zur Phthalsäureanhydridherstellung integrierten erfindungsgemäßen Reaktors;
- Figur 2: einen Längsschnitt einer ersten Ausführungsform des erfindungsgemäßen Reaktors;
- Figur 3: einen Querschnitt durch den Reaktor der Fig. 2 entlang der Linie III - III;
- Figur 4: einen vergrößerten Detailausschnitt des Reaktors der Figur 3;
- Figur 5: einen Längsschnitt einer zweiten Ausführungsform des erfindungsgemäßen Reaktors;
- Figur 6: einen Querschnitt durch den Reaktor der Fig. 5 entlang der Linie VI - VI;
- Figur 7: einen Querschnitt durch eine dritte Ausführungsform des erfindungsgemäßen Reaktors;und
- Figur 8: einen Längsschnitt einer vierten Ausführungsform des erfindungsgemäßen Reaktors.

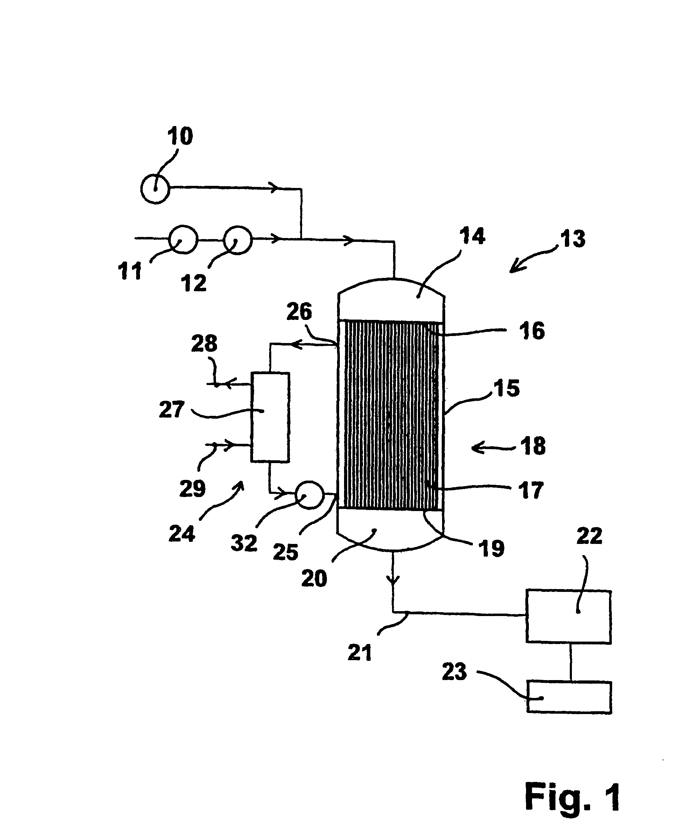

Bezugnehmend auf Figur 1 erkennt man eine schematisch dargestellte Anlage zur Phthalsäureanhydrid-Herstellung. Eine detaillierte Beschreibung des Herstellungsverfahrens findet sich beispielsweise in Ullmann's Encyclopedia of Industrial Chemistry, 5^{th} Edition, Vol. 20A, S. 181 ff.

In einem Verdampfer 10 wird o-Xylol oder Naphthalin verdampft und mit einem Überschuß an durch ein Gebläse 11 und einen Erhitzer 12 auf ca. 120 bis 300° C erhitzter Luft gemischt. Das o-Xylol/Luft-Gemisch gelangt in den Reaktor 13, wo es im Bereich der oberen Haube 14 gleichmäßig über den gesamten Reaktorquerschnitt verteilt wird. Die obere Haube 14 wird zum zylindrischen Reaktormantel 15 hin durch ein oberes Bodenblech 16 abgeschlossen. In das Bodenblech 16 münden die Reaktionsrohre 17 des Rohrbündels 18. Die Reaktionsrohre 17 sind in ihrem oberen Bereich abdichtend mit dem Boden 16 verschweißt. In den Reaktionsrohren 17 befindet sich das (nicht dargestellte) Katalysatormaterial. In ihrem unteren Bereich sind die Reaktionsrohre 17 mit einem unteren Bodenblech 19 abdichtend verschweißt und münden in eine untere Haube 20 des Reaktors 13. Das o-Xylol/Luft-Gemisch durchströmt die Reaktionsrohre und wird größtenteils zu Phthalsäureanhydrid oxidiert. Das heiße Reaktionsgas wird über eine Leitung 21 zu sog. Desublimatoren oder Abscheidern 22 geleitet, wo es in Form feinster Kristalle abgeschieden wird. Von den Abscheidern 22 wird das Phthalsäureanhydrid abgeschmolzen und in einer anschließenden Destillationseinrichtung 23 aus dem abgeschmolzenen Roh-Phthalsäureanhydrid die Reinsubstanz gewonnen (entsprechend Ullmann's Encyclopedia of Industrial Chemistry, 5th Edition, Vol. 20A, S. 181 ff.).

Das Reaktionsrohrbündel 18 wird durch einen Wärmetauschmittelkreislauf, der insgesamt mit der Bezugsziffer 24 bezeichnet ist, temperiert. Dazu wird eine Salzschmelze aus Natriumnitrat, Natriumnitrit und Kaliumnitrit über Mantelöffnungen 25 in den zylindrischen Mantelabschnitt des Reaktors geleitet und dort im Längs-, Kreuzquer-, Gegen- oder Gleichstrom an den Reaktionsrohren 17 des Bündels 18 vorbeigeführt, um die bei der Oxidation von o-Xylol entstehende Reaktionswärme abzuführen.

Das Wärmetauschmittel verläßt den Reaktor über Mantelöffnungen 26 und gelangt in einen außenliegenden Wärmetauscher 27, der von einem (nicht dargestellten) Dampferzeuger über Leitungen 28, 29 auf die gewünschte Reaktionstemperatur, die meist in einem Bereich zwischen 340 und 440 °C liegt, temperiert wird. Die genaue Wahl der Reaktionstemperatur hängt insbesondere vom verwendeten Katalysatormaterial ab und sollte möglichst konstant eingehalten werden.

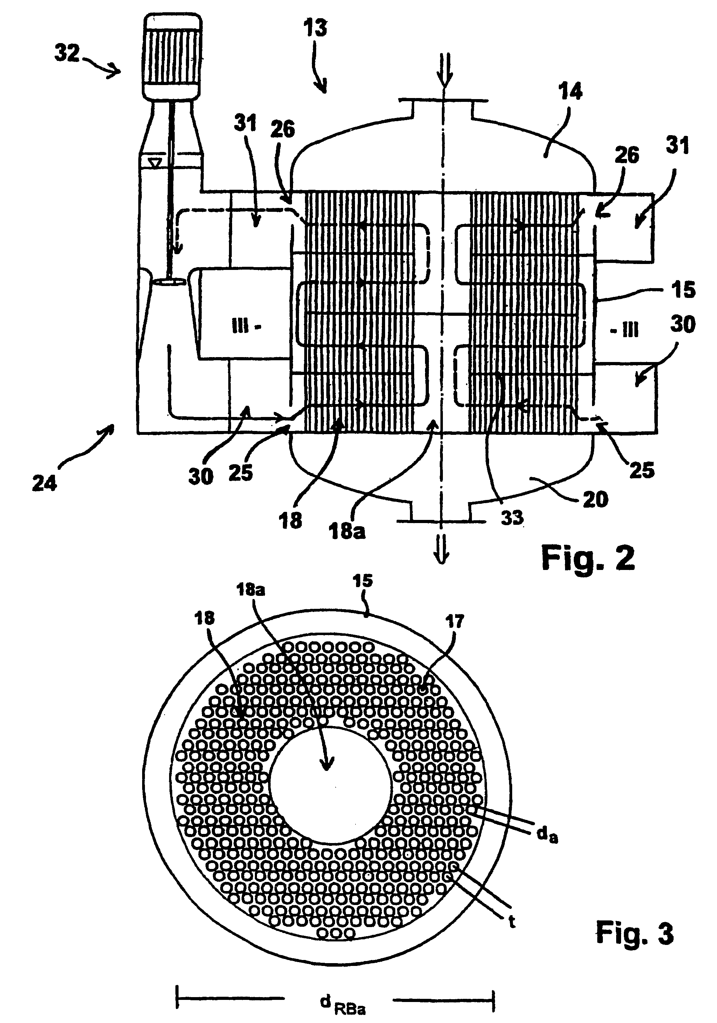

In Figur 2 ist eine erste Ausführungsform des erfindungsgemäßen Rohrbündelreaktors detaillierter dargestellt. Figur 3 zeigt einen Schnitt durch den in Figur 2 dargestellten Reaktor entlang der Linie III - III. Elemente, welche eine vergleichbare Funktion wie bereits im Zusammenhang mit Figur 1 beschriebene Elemente erfüllen, sind mit den gleichen Bezugsziffern bezeichnet.

Der zylindrische Reaktor 13 weist ein vertikal angeordnetes Reaktionsrohrbündel 18 mit kreisförmigem Querschnitt und einem Außendurchmesser *d*_{*RBa*} auf (vergl. Fig. 3). Die Reaktionsrohre 17 sind auf einem Kreisring gleichmäßig verteilt. Der mittlere Bereich 18a des Rohrbündels 18 ist Reaktionsrohrfrei.

Das Wärmetauschmittel wird über eine oder mehrere Pumpen 32 über Ringleitungen 30,31 durch Mantelöffnungen 25,26 dem die Reaktionsrohre umgebenden Raum zu- bzw. aus diesem abgeführt. Mit Hilfe von in dem Reaktor angeordneten Umlenkscheiben 33 wird eine meanderförmige Strömung des Wärmetauschmittels realisiert, wobei jedoch im Bereich der Reaktionsrohre 17 eine radiale Strömung vorherrscht.

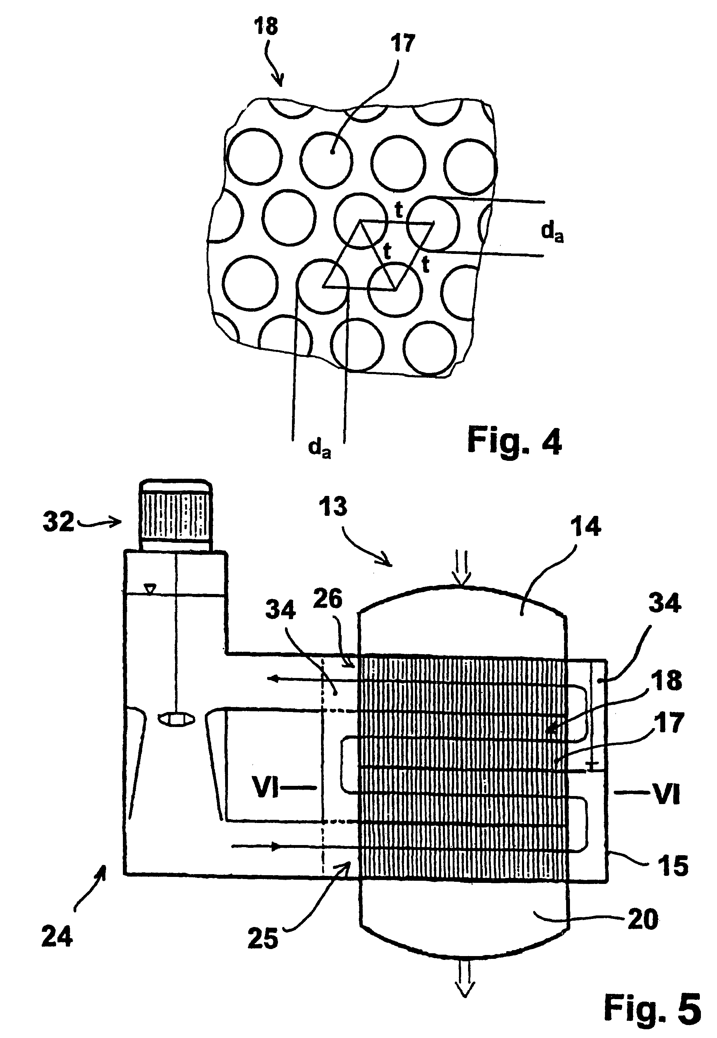

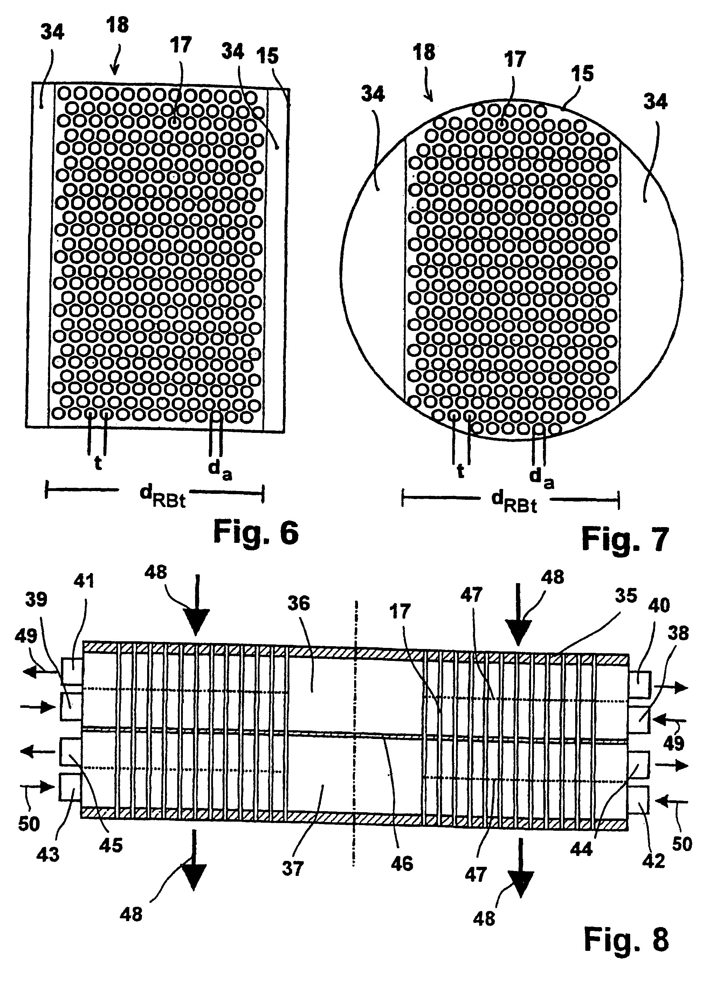

In der Querschnittsdarstellung der Figuren 3 und 4 sind die Rohrteilung *t*, der Außendurchmesser *d*_{*a*} eines Reaktionsrohrs 17 und der Außendurchmesser *d*_{*RBa*} des Reaktionsrohrbündels 18 angedeutet. Es sei angemerkt, daß die Darstellung der Figur 3 (ebso wie die der Figuren 6 und 7) nicht maßstäblich ist. In der Realität ist der Durchmesser der Reaktionsrohre 17 verglichen dem Rohrbündelaußendurchmesser wesentlich kleiner.

Zur besseren Veranschaulichung der wesentlichen Kenngrößen des erfindungsgemäßen Rohrbündelreaktors ist dabei in Figur 4 ein Ausschnitt des in Figur 3 gezeigten Rohrbündels 18 in größerem Maßstab dargestellt.Insbesondere in der Darstellung der Fig. 4 erkennt man, daß jeweils drei benachbarte Reaktionsrohre die Eckpunkte eines gleichseitigen Dreiecks bilden.

Die in Figuren 5 und 6 dargestellte Ausführungsform des erfindungsgemäßen Reaktors, sowie die Variante der Figur 7 weisen jeweils ein Reaktionsrohrbündel 18 mit im wesentlichen rechteckigem Querschnitt auf. Mit einer solchen Geometrie sind insbesonders bei einer Querstromkühlung Vorteile aufgrund von geringeren Strömungswiderständen für den Wärmetauschmitteltransport verbunden.

Mit dem erfindungsgemäß vorgeschlagenen Teilungsverhältnis werden dieser Vorteile weiter verstärkt.

An einander gegenüber liegenden breiten Seitenflächen des Reaktors sind reaktionsrohrfreie Räume 34 zum Verteilen bzw. Sammeln des Wärmetauschmittels vorgesehen. Bei der Variante der Figur 6 hat der Reaktormantel 15 selbst einen rechteckigen Querschnitt, während bei der Variante der Figur 7 ein zylindrischer Reaktor eingesetzt wird. Der im wesentlichen rechteckige Querschnitt des Reaktionsrohrbündels 18 entsteht im letzteren Fall durch die reaktionsrohrfreien Rohrsegmente 34. Erfindungsgemäß wird im Fall von im wesentlichen rechteckigen Reaktionsrohrbündeln das Teilungsverhältnis in Abhängigkeit von der quer durchströmten Tiefe *d*_{*RBt*} des Rohrbündels 18 gewählt.

In Figur 8 ist schließlich eine vierte Ausführungsform des erfindungsgemäßen Rohrbündelreaktors in einem schematischen Längsschnitt dargestellt. Bei dem Reaktor 35 handelt es sich in diesem Fall um einen Zweizonenreaktor, der, in Längsrichtung der Reaktionsrohre 17 betrachtet, in zwei unterschiedlich temperierte Zonen, 36, 37 unterteilt ist. Die Zonen 36, 37 werden von separaten Wärmetauschmittelkreisläufen versorgt. Im Beispiel wird eine erste Salzlösung über die Stutzen 38, 39 in die erste Zone 36 eingeleitet und über Stutzen 40, 41 wieder abgeführt. Entsprechend wird eine zweite Salzlösung über Stutzen 42, 43 in die zweite Zone geleitet und über Stutzen 44, 45 wieder abgeführt. Die beiden Zonen 36, 37 sind über ein 50 mm dickes Rohrbodenblech 46 voneinander getrennt. Das Blech weist Ausnehmungen auf, durch welche die Reaktionsrohre 17 eingesetzt werden. Nach Einsetzen der Rohre werden diese hydraulisch etwas erweitert, so daß ein guter und weitgehend dichter Sitz der Rohre 17 im Rohrbodenblech 46 erreicht wird. Innerhalb einer Zone sind Umlenkbleche 47 angeordnet, welche die Salzschmelze radial von Außen in ein reaktionsrohrfreies Zentrum des Reaktors leiten, wo sie nach oben umgelenkt und wieder radial nach Außen abgeführt wird. In Figur 8 ist mit großen Pfeilen 48 die Strömungsrichtung der Reaktionsgase und mit kleineren Pfeilen 49, 50 der Strom der ersten bzw. zweiten Salzschmelze symbolisiert.

### Beispiele

Die in den im folgenden beschriebenen Beispielen verwendeten Katalysatoren wurden wie folgt hergestellt.

### Katalysator I:

50 kg Steatit (Magnesiumsilikat)-Ringe mit einem äußeren Durchmesser von 8 mm, einer Länge von 6 mm und einer Wandstärke von 1,5 mm wurden in einer Dragiertrommel auf 160 °C erhitzt und mit einer Suspension aus 28,6 kg Anatas mit einer BET-Oberfläche von 20 m²/g, 2,19 kg Vanadyloxalat, 0,176 kg Cäsiumsulfat, 44,1 kg Wasser und 9,14 kg Formamid besprüht, bis das Gewicht der aufgetragenen Schicht 10,5% des Gesamtgewichts des fertigen Katalysators betrug (nach Calcination bei 450 °C).
Die auf diese weise aufgebrachte katalytisch aktive Masse, also die Katalysatorschale, bestand aus 4,0 Gew.-% Vanadium (berechnet als V₂O₅)i 0,4 Gew.-% Cäsium (berechnet als Cs) und 95,6 Gew.-% Titandioxid.

### Katalysator II:

50 kg Steatit (Magnesiumsilikat)-Ringe mit einem äußeren Durchmesser von 8 mm, einer Länge von 6 mm und einer Wandstärke von 1,5 mm wurden in einer Dragiertrommel auf 160 °C erhitzt und mit einer Suspension aus 28,6 kg Anatas mit einer BET-Oberfläche von 20 m²/g, 4,11 kg Vanadyloxalat, 1,03 kg Antimontrioxid, 0,179 kg Ammoniumdihydrogenphosphat, 0,045 kg Cäsiumsulfat, 44,1 kg Wasser und 9,14 kg Formamid besprüht, bis das Gewicht der aufgetragenen Schicht 10,5% des Gesamtgewichts des fertigen Katalysators betrug (nach Calcination bei 450 °C).
Die auf diese weise aufgebrachte katalytisch aktive Masse, also die Katalysatorschale, bestand aus 0,15 Gew.-% Phosphor (berechnet als P), 7,5 Gew.-% Vanadium (berechnet als V₂O₅), 3,2 Gew.-% Antimon (berechnet als Sb₂O₃), 0,1 Gew.-% Cäsium (berechnet als Cs) und 89,05 Gew.-% Titandioxid.

### Beispiel 1: Herstellung von PSA mit einem erfindungsgemäßen Reaktor.

In dem erfindungsgemäßen Reaktor war ein Rohrbündel mit einem Außendurchmesser von *d*_{*RBa*} = *5,435 m* angeordnet. Es bestand aus ca. 14.000 Reaktionsrohren aus Stahl, die jeweils 3,5 m lang waren und einen Außendurchmesser *d*_{*a*} *= 29 mm* hatten. Die Rohrteilung *t* betrug 40 mm; somit war das Verhältnis *t*/*d*_{*a*} = *1,3793*. Durch das Rohr wurden stündlich von oben nach unten 4 Nm³ Luft mit einer Beladung an 98,5 Gew.-%igem o-Xylol von 90 g/Nm³ geleitet. Die Reaktionsrohre waren so gefüllt, daß zwei unterschiedlich aktive Katalysatorzonen gebildet wurden. Dazu wird zunächst der Katalysator II in jedes Rohr gefüllt, bis eine (vom Boden aus gemessene) Schüttungshöhe von 1,3 m erreicht wurde. Dann wird eine Schüttung des Katalysators I mit einer Höhe von 1,7 m auf die Schicht des Katalysator II aufgebracht.

Als Wärmetauschmittel wurde eine Salzschmelze aus KNO₃,NaNO₂ und NaNO₃ verwendet, die mit einer Temperatur von 348,9 °C bei einem Durchfluß von 11.000 m³ pro Stunde durch den Reaktor geleitet wurde. Die Austrittstemperatur der Schmelze betrug 351,1 °C. Die Hotspot-Temperatur der Salzschmelze lag 3,98 °C über der Salzeintrittstemperatur.

Auf verschiedenen Querschnittsniveaus waren radial mehrere Temperaturfühler in der Schmelze angeordnet. Die gemessenen Temperaturunterschiede über den Reaktorquerschnitt betrugen maximal ca. 2,2 °C.

Die Ausbeute an ans PSA betrug 78.9 mol%.

### Beispiel 2: (Vergleichsbeispiel) Herstellung von PSA mit einem Reaktor gemäß Stand der Technik.

In dem Reaktor des Standes der Technik war ein Rohrbündel mit einem Außendurchmesser von *d*_{*RBa*} *= 5,021 m* angeordnet. Es bestand aus ca. 14.000 Reaktionsrohren aus Stahl, die jeweils 3,5 m lang waren und einen Außendurchmesser *d*_{*a*} = *30 mm* hatten. Die Rohrteilung t betrug 38 mm; somit war das Verhältnis *t*/*d*_{*a*} *= 1,267.* Wieder wurden durch die Füllung der Reaktionsrohre - wie in Beispiel 1 beschrieben - zwei unterschiedlich aktive Katalysatorzonen gebildet.

Durch das Rohr wurden wieder stündlich von oben nach unten 4 Nm³ Luft mit einer Beladung an 98,5 Gew.-%igem o-Xylol von 90 g/Nm³ geleitet. Als Wärmetauschmittel wurde wie im erfindungsgemäßen Beispiel eine Salzschmelze aus KNO₃,NaNO₂ und NaNO₃ verwendet.

Die Eintrittstemperatur der Schmelze betrug 345,9 °C; es wurden 6.200 m³ Schmelze pro Stunde durch den Reaktor geleitet. Die Austrittstemperatur der Schmelze lag bei 349,7 °C. Die Hotspot-Temperatur der Salzschmelze lag 7,2 °C über der Salzeintrittstemperatur.

Die gemessenen Temperaturunterschiede über den Reaktorquerschnitt betrugen maximal ca. 4,2 °C.

Die Ausbeute an PSA betrug 77,8 mol%.

## Patentansprüche

1. Rohrbündelreaktor (13) mit einem in einem Mantel (15) angeordneten, aus zahlreichen parallelen Reaktionsrohren (17) bestehenden Reaktionsrohrbündel (18), wobei das Reaktionsrohrbündel (18) 10.000 bis 50.000 Reaktionsrohre (17) aufweist, und mit Mitteln zur Zu- und Abführung eines die Reaktionsrohre (17) umströmenden Wärmetauschmittels, wobei die Reaktionsrohre einen Außendurchmesser *d*_{*a*} und eine Rohrteilung *t* auf weisen, **dadurch gekennzeichnet, daß**
das Verhältnis *t* / *d*_{*a*} von Rohrteilung *t* zum Außendurchmesser *d*_{*a*} eines Reaktionsrohrs wenigstens 1,3 beträgt.

2. Rohrbündelreaktor gemäß Anspruch 1, **dadurch gekennzeichnet, daß** das Verhältnis *t*/*d*_{*a*} von Rohrteilung *t* zum Außendurchmesser *d*_{*a*} eines Reaktionsrohrs (17) mit zunehmenden Querabmessungen des Reaktionsrohrbündels (18) ansteigt.

3. Rohrbündelreaktor gemäß einem der Ansprüche 1 oder 2, **dadurch gekennzeichnet, daß** das Reaktionsrohrbündel (18) einen im wesentlichen kreisförmigen Querschnitt mit einem Außendurchmesser *d*_{RBa} von mehr als 4 m aufweist.

4. Rohrbündelreaktor gemäß Anspruch 3, **dadurch gekennzeichnet, daß** der Außendurchmesser *d*_{RBa} des Reaktionsrohrbündels (18) zwischen 4 m und 12 m beträgt und das Verhältnis *t*/*d*_{*a*} von Rohrteilung t zum Außendurchmesser *d*_{*a*} eines Reaktionsrohrs (17) im Bereich von 1,3 und 1,6 liegt.

5. Rohrbündelreaktor gemäß Anspruch 4, **dadurch gekennzeichnet, daß** der Außendurchmesser *d*_{RBa} des Reaktionsrohrbündels (18) zwischen 4 m und 10 m beträgt und das Verhältnis *t*/*d*_{*a*} von Rohrteilung *t* zum Außendurchmesser *d*_{*a*} eines Reaktionsrohrs (17) im Bereich von 1,3 und 1,5 liegt.

6. Rohrbündelreaktor gemäß einem der Ansprüche 1 oder 2, **dadurch gekennzeichnet, daß** das Reaktionsrohrbündel (18) einen im wesentlichen rechteckigen Querschnitt mit einer parallel zur Strömungsrichtung des Wärmetauschmittels gemessenen Rohrbündeltiefe *d*_{*RBt*} von wenigstens 1,3 m aufweist.

7. Rohrbündelreaktor gemäß Anspruch 6, **dadurch gekennzeichnet, daß** die Tiefe *d*_{*RBt*} des Reaktionsrohrbündels (18) zwischen 1,3 und 4 m beträgt und das Verhältnis *t*/*d*_{*a*} von Rohrteilung *t* zum Außendurchmesser *d*_{*a*} eines Reaktionsrohrs (17) im Bereich von 1,3 und 1,6 liegt.

8. Rohrbündelreaktor gemäß einem der Ansprüche 1 bis 7, **dadurch gekennzeichnet, daß** der Reaktor in Längsrichtung der Reaktionsrohre (17) in mindestens zwei Zonen (36,37) unterteilt ist, die von verschieden temperiertem Wärmetauschmittel durchströmt werden.

9. Verwendung eine Rohrbündelreaktors nach einem der Ansprüche 1 bis 8 zur Durchführung von katalytischen Gasphasenreaktionen.

10. Verwendung eines Rohrbündelreaktors nach einem der Ansprüche 1 bis 8 zur Durchführung von Oxidationsreaktionen, insbesondere zur Herstellung von Phthalsäureanhydrid, Maleinsäureanhydrid, Acrylsäure, Acrolein, Methacrylsäure, Glyoxal, Phosgen, Blausäure oder Vinylformamid.

## Claims

1. A multitube reactor (13) which has a catalyst tube bundle (18) comprising numerous parallel catalyst tubes (17) arranged within an outer wall (15), said catalyst tube bundle (18) having from 10,000 to 50,000 catalyst tubes (17), and having means for introducing and discharging a heat transfer medium flowing around the catalyst tubes (17), said catalyst tubes having an external diameter *d*_{*a*} and a tube spacing *t*, **characterized in that** the ratio *t*/*d*_{*a*} of tube spacing *t* to the external diameter *d*_{*a*} of a catalyst tube is at least 1.3

2. A multitube reactor as claimed in claim 1, **characterized in that** the ratio *t*/*d*_{*a*} of tube spacing *t* to the external diameter *d*_{*a*} of a catalyst tube (17) rises with increasing transverse dimensions of the catalyst tube bundle (18).

3. A multitube reactor as claimed in claim 1 or 2, **characterized in that** the catalyst tube bundle (18) has an essentially circular cross section having an external diameter *d*_{*RBa*} of more than 4 m.

4. A multitube reactor as claimed in claim 3, **characterized in that** the external diameter *d*_{*RBa*} of the catalyst tube bundle (18) is from 4 m to 12 m and the ratio *t*/*d*_{*a*} of tube spacing *t* to the external diameter *d*_{*a*} of a catalyst tube (17) is in the range from 1.3 to 1.6.

5. A multitube reactor as claimed in claim 4, **characterized in that** the external diameter *d*_{*RBa*} of the catalyst tube bundle (18) is from 4 m to 10 m and the ratio *t*/*d*_{*a*} of tube spacing *t* to the external diameter *d*_{*a*} of a catalyst tube (17) is in the range from 1.3 to 1.5.

6. A multitube reactor as claimed in claim 1 or 2, **characterized in that** the catalyst tube bundle (18) has an essentially rectangular cross section with a tube bundle depth *d*_{*RBt*} measured parallel to the flow direction of the heat transfer medium of at least 1.3 m.

7. A multitube reactor as claimed in claim 6, **characterized in that** the depth *d*_{*RBt*} of the catalyst tube bundle (18) is from 1.3 to 4 m and the ratio *t*/*d*_{*a*} of tube spacing *t* to the external diameter *d*_{*a*} of a catalyst tube (17) is in the range from 1.3 to 1.6.

8. A multitube reactor as claimed in any of claims 1 to 7, **characterized in that** the reactor is devided, in the longitudinal direction of the catalyst tubes (17), into at least two zones (36,37), with a flow of heat transfer medium of different temperature being provided in each zone.

9. The use of a multitube reactor as claimed in any of claims 1 to 8 for carrying out catalytic gas-phase reactions.

10. The use of a multitube reactor as claimed in any of claims 1 to 8 for carrying out oxidation reactions, in particular for the preparation of phthalic anhydride, maleic anhydride, acrylic acid, acrolein, methacrylic acid, glyoxal, phosgene, hydrocyanic acid or vinyl formamide.

## Revendications

1. Réacteur à faisceau tubulaire (13) présentant un faisceau de tubes de réaction (18) composé d'une pluralité de tubes de réaction parallèles (17) disposés dans une gaine (15), le faisceau de tubes de réaction (18) présentant 10 000 à 50 000 tubes de réaction parallèles (17), et le réacteur comprenant des moyens permettant l'acheminement et de l'évacuation d'un agent caloporteur circulant autour des tubes de réaction (17), les tubes de réaction présentant un diamètre externe dₑ et une section de tube t, **caractérisé en ce que** le rapport t/dₑ de la section de tube t au diamètre externe dₑ est d'au moins 1,3.

2. Réacteur à faisceau tubulaire selon la revendication 1, **caractérisé en ce que** le rapport t/dₑ de la section de tube t au diamètre externe dₑ d'un tube de réaction (17) augmente proportionnellement à la section transversale du faisceau de tubes de réaction (18).

3. Réacteur à faisceau tubulaire selon la revendication 1 ou 2, **caractérisé en ce que** le faisceau de tubes de réaction (18) présente une section transversale essentiellement circulaire ayant un diamètre externe Fdₑ supérieur à 4 m.

4. Réacteur à faisceau tubulaire selon la revendication 3, **caractérisé en ce que** le diamètre externe Fdₑ du faisceau de tubes de réaction (18) mesure entre 4 m et 12 m et que le rapport t/dₑ de la section de tube t au diamètre externe dₑ d'un tube de réaction (17) va de 1,3 à 1,6.

5. Réacteur à faisceau tubulaire selon la revendication 4, **caractérisé en ce que** le diamètre externe Fdₑ du faisceau de tubes de réaction (18) mesure entre 4 m et 10 m et que le rapport t/dₑ de la section de tube t au diamètre externe de d'un tube de réaction (17) va de 1,3 à 1,5.

6. Réacteur à faisceau tubulaire selon l'une quelconque des revendications 1 ou 2, **caractérisé en ce que** le faisceau de tubes de réaction (18) présente une section transversale essentiellement rectangulaire présentant une profondeur du faisceau de tubes Fₚ mesurée dans le sens parallèle à la direction d'écoulement de l'agent caloporteur d'au moins 1,3 m.

7. Réacteur à faisceau tubulaire selon la revendication 6, **caractérisé en ce que** la profondeur du faisceau de tubes de réaction (18) Fₚ va de 1,3 m à 4 m et que le rapport t/dₑ de la section t au diamètre externe d'un tube de réaction (17) va de 1,3 à 1,6.

8. Réacteur à faisceau tubulaire selon l'une quelconque des revendications 1 à 7, **caractérisé en ce que** le réacteur est subdivisé dans le sens longitudinal des tubes de réaction (17) en au moins deux zones (36, 37), sont traversées par des agents caloporteurs chauffés à des températures différentes.

9. Utilisation d'un réacteur à faisceau tubulaire selon l'une quelconque des revendications 1 à 8 en vue de la réalisation de réactions catalytiques en phase gazeuse.

10. Utilisation d'un réacteur à faisceau tubulaire selon l'une quelconque des revendications 1 à 9 en vue de la réalisation de réactions d'oxydation, en particulier pour la préparation d'anhydride phtalique, d'anhydride maléique, d'acide acrylique, d'acroléine, d'acide méthacrylique, de glyoxal, de phosgène, d'acide cyanhydrique ou de vinylformamide.
